# EUROPEAN PATENT APPLICATION

(11) **EP 2 805 627 A1**
(43) Date of publication of application: **26.11.2014**
(21) Application number: 14186390.2
(22) Date of filing: 25.09.2014
(51) Int. Cl.: A23L 1/308, A23G 3/42, A23K 1/16, A23L 1/09, A23L 1/10, A61K 31/716, A23L 2/52

(54) **Carbohydrate compositions**

(71) Applicant: Cargill, Incorporated, Wayzata, MN 55391 (US)
(72) Inventor: De Sadeleer, Jos, 3220 Holsbeek (BE); Pollet, Annick, 3271 Zichem (BE)
(74) Representative: Kiadi Matsuela, Dela

(57) **Abstract**

The present invention relates to a carbohydrate mixture comprising a first fiber containing material and a second fiber containing material wherein the first fiber containing material is wheat bran derived arabinoxylan-oligosaccharides having an average degree of polymerisation of at least 2 to 100 and optionally wheat bran derived xylo-oligosaccharides having an average degree of polymerisation of at least 2 to 100, and wherein the second fiber containing carbohydrate based material is endosperm derived xylo-oligosaccharides and arabinoxylan-oligosaccharides having an average degree of polymerisation of at least 50 to 100 and/or hydrogenated endosperm derived xylo-oligosaccharides and arabinoxylanoligosaccharides having an average degree of polymerisation of at least 50 to 100; and wherein the wheat bran derived arabinoxylan-oligosaccharides and xylo-oligosaccharides are soluble in water.

## Description

### Field of the Invention

The present invention relates to a carbohydrate mixture that provides fiber into food, pet food, feed, and beverages.

### Background of the Invention

An increasing preoccupation of consumers with nutrition and the potential beneficial effects of foods is nowadays observed. For many years great efforts have been made to replace the high-calorie substances in traditional foods as means of avoiding or reducing risks of chronic diseases like obesity, vascular and heart diseases, constipation, and/or reducing risk for colon cancer and the like.

As is generally known, carbohydrates represent one of the essential foundations of nutrition. The task of the carbohydrates therefore is primarily of the nutritive kind and where possible to serve as fiber respectively.

Due to the variability of the monomers (being mainly C5 or C6 monomers) forming the oligo and polysaccharides, and due to the position of the glycosidic bond and the anomeric state of the carbohydrates, they represent a large and heterogeneous class of different products. Several types of carbohydrates mixtures and derivatives have been proposed to provide nutrition and/or fiber content.

However, there is still a further need of providing suitable carbohydrate mixtures with health benefits. The current invention provides such a carbohydrate mixture.

### Summary of the Invention

The current invention relates to a carbohydrate mixture comprising a first fiber containing material and a second fiber containing material wherein the first fiber containing material is wheat bran derived arabinoxylan-oligosaccharides having an average degree of polymerisation of at least 2 to 100 and optionally wheat bran derived xylo-oligosaccharides having an average degree of polymerisation of at least 2 to 100, and wherein the second fiber containing carbohydrate based material is endosperm derived xylo-oligosaccharides and arabinoxylan-oligosaccharides having an average degree of polymerisation of at least 50 to 100 and/or hydrogenated endosperm derived xylo-oligosaccharides and arabinoxylan-oligosaccharides having an average degree of polymerisation of at least 50 to 100.

The current invention further relates to the use of carbohydrate mixture of current invention in compositions intended to be ingested by humans and animals.

### Detailed Description

The current invention relates to a carbohydrate mixture comprising a first fiber containing material and a second fiber containing material wherein the first fiber containing material is wheat bran derived arabinoxylan-oligosaccharides having an average degree of polymerisation of at least 2 to 100 and optionally wheat bran derived xylo-oligosaccharides having an average degree of polymerisation of at least 2 to 100, and wherein the second fiber containing carbohydrate based material is endosperm derived xylo-oligosaccharides and arabinoxylan-oligosaccharides having an average degree of polymerisation of at least 50 to 100 and/or hydrogenated endosperm derived xylo-oligosaccharides and arabinoxylan-oligosaccharides having an average degree of polymerisation of at least 50 to 100.

### Dietary fiber

Dietary fiber (fiber in the current invention) refers to indigestible carbohydrate based material. It encompasses carbohydrate polymers which are not hydrolyzed by the endogenous enzymes in the human small intestine. It may encompass two types of fibers. Either the fiber is soluble in water or it refers to a fiber which is not soluble in water. Soluble fibers in the sense of the current invention are understood those, which form a homogeneous solution in water, in a concentration of at least 1 g/L at room temperature.

Many of the physiological effects associated with dietary fiber consumption, including effects on stool parameters, colonic health, mineral absorption in the colon, vitamin synthesis in the colon, the metabolic syndrome and immune system, are believed to promote health and wellbeing of the consumer. Dietary fibers have a strong impact on the colonic ecosystem and serve as primary substrates for intestinal microbiota metabolism.

### Wheat bran derived AXOS

Wheat bran derived arabinoxylan-oligosaccharides (AXOS) are characterized by an average degree of polymerisation (DP). DP is the number of monomeric units forming the oligosaccharide chain. The term "wheat bran derived arabinoxylan-oligosaccharides", in the context of the present invention, refers to preparations derived from wheat bran and consisting predominantly of arabinoxylan-oligosaccharides and minor amounts of xylo-oligosaccharides and corresponding to poly- or oligosaccharides comprising a backbone of beta-(1-4)-linked D-xylopyranosyl residues (xylose) with an average DP from 2 and 100, preferably from 2 to 80, 2 to 70, 2 to 60, 2 to 50, 3 to 30, 3 to 20, 5 to 10, 4 to 8, 3 to 6 with no (in the case of xylo-oligosaccharides) or at least one alpha-L-arabinofuranosyl (arabinose) residue linked to one of the xylose residues of the backbone per molecule. Other substituents such as acetyl, alfa-glucuronyl, alfa-4-O-methylglucuronyl, galacturonyl, xylosyl, rhamnosyl, galactosyl, or glucosyl side chains, or short oligosaccharide side chains, can be attached to one or more of the xylose residues and hydroxycinnamic acids, such as ferulic acid, dehydrodiferulic acid, p-coumaric acid, caffeic acid or sinapic acid, can be linked to one or more of the arabinose units. Wheat bran derived AXOS can be derived from arabinoxylans by partial hydrolysis, e.g. using acids or arabinoxylan degrading enzymes and/or physical treatment.

Wheat bran derived AXOS are further characterized by their average degree of arabinose substitution (arabinose to xylose ratio, A/X ratio). Considering the large influence of carbohydrate structure on fermentation properties, different average degrees of arabinose substitution has different effects on the health of the human or animal consuming it. Preferably wheat bran derived AXOS having an average degree of arabinose substitution of 0.1 to 0.7, such as: 0.1 to 0.3, 0.1 to 0.2, 0.17 to 0.25, 0.2 to 0.3, 0.3 to 0.4, 0.4 to 0.5, 0.5 to 0.6 or 0.6 to 0.7, can be produced.

Wheat bran derived AXOS are further characterized by the presence of other substituents such as ferulic acid. Ferulic acid and feruloylated wheat bran derived AXOS have antioxidant properties (Ou et al. 2007, Journal of agricultural and food chemistry 55 (8), 3191-3195). Preferably, wheat bran derived AXOS having a ferulic acid content of 1 to 10 w/w %, such as 1 to 3 w/w %, 2 to 3 w/w %, 3 to 4 w/w %, 4 to 5 w/w %, 5 to 6 w/w %, 6 to 7 w/w %, 7 to 8 w/w %, 8 to 9 w/w %, 9 to 10 w/w %.

Wheat bran derived AXOS are further characterized by their molecular weight distribution.

Different average DP provide different properties to wheat bran derived AXOS: those having a low average DP, ranging from 3 to 50 have been shown to provide prebiotic effects, those having high average DP, above 50, can also have an impact on the viscosity, the mouthfeel and texture of the composition where they are present.

It is further to be noted that wheat bran derived AXOS in the present invention also covers the preparations that are not 100% pure and are further containing ashes, proteins and other carbohydrates. It goes without saying that the current invention also covers the wheat bran derived AXOS enriched preparations whereby wheat bran derived AXOS can be added in liquid or powder form.

### Endosperm derived xylo-oligosaccharides and arabinoxylan-oligosaccharides

Endosperm derived xylo-oligosaccharides and arabinoxylan-oligosaccharides having an average degree of polymerisation of from 50 to 100, preferably from 60 to 100. These Endosperm derived xylo-oligosaccharides and arabinoxylan-oligosaccharides are obtainable by partial hydrolysis of the endosperm arabinoxylans, e.g. using either acids or endoxylan degrading enzymes and/or physical treatment. Without being limited, a suitable preparation of endosperm derived xylo-oligosaccharides and arabinoxylan-oligosaccharides is for example provided in WO 2010/020639. Hydrogenated endosperm derived xylo-oligosaccharides and arabinoxylan-oligosaccharides are prepared by hydrogenating the endosperm derived xylo-oligosaccharides and arabinoxylan-oligosaccharides.

Surprisingly it is found that by applying as first fiber-containing material wheat bran derived AXOS and by combining it with the second fiber-containing material mentioned in claim 1, the effects, and in particular the health benefits have been synergistically improved.

The advantages of the carbohydrate mixture of the current invention are preferably demonstrated where the carbohydrate mixture is containing as first fiber containing material the wheat bran derived arabinoxylan-oligosaccharides and optionally wheat bran derived xylo-oligosaccharides, having a degree of polymerisation of from 2 to 50.

Furthermore, the current invention relates to the carbohydrate mixture of the current invention wherein the first fiber containing material is present in an amount of from 5 to 95% based on dry matter content of the carbohydrate mixture. It further relates to the carbohydrate mixture of the current invention wherein the first fiber containing material is present in an amount of from 10 to 90% based on dry matter content of the carbohydrate mixture. It further relates to the carbohydrate mixture of the current invention wherein the first fiber containing material is present in an amount of from 20 to 80% based on dry matter content of the carbohydrate mixture. It further relates to the carbohydrate mixture of the current invention wherein the first fiber containing material is present in an amount of from 25 to 75% based on dry matter content of the carbohydrate mixture. It further relates to the carbohydrate mixture of the current invention wherein the first fiber containing material is present in an amount of from 30 to 70% based on dry matter content of the carbohydrate mixture. It further relates to the carbohydrate mixture of the current invention wherein the first fiber containing material is present in an amount of from 40 to 60% based on dry matter content of the carbohydrate mixture. It further relates to the carbohydrate mixture of the current invention wherein the first fiber containing material is present in an amount of about 50% based on dry matter content of the carbohydrate mixture.

The current invention relates to the to the carbohydrate mixture of the current invention wherein the first fiber containing material is present in an amount of from 5 to 95% based on dry matter content of the carbohydrate mixture and the second fiber containing carbohydrate based material is present in an amount of 5 to 95% based on dry matter content of the carbohydrate mixture. The second fiber containing carbohydrate based material may be present in an amount of 10%, 20%, 25%, 30%, 40%, 50%, 60%, 70%, 75%, 80% or 90% based upon the dry matter content of the carbohydrate mixture.

The inventors find that foods, pet food, feed (suitable for non-ruminants (i.e. monogastric animals, poultry and aqua) and beverages containing the carbohydrate mixture of the current invention added thereto or food, pet food, feed, and beverages partially replaced with the carbohydrate mixture of the current invention exhibit various health benefits which contribute to the prevention of diseases caused by the living habits. Surprisingly, it is seen that the carbohydrate mixture of the current invention has impacted synergistically the health benefits in comparison to each fiber containing carbohydrate based materials applied alone or to the second fiber containing carbohydrate based materials applied alone.

The health benefits can be explicitly exhibited when the daily intake of the carbohydrate mixture of the current invention is at least amounting in total to 1 g, preferably 1.5 g, preferably to at least 2.5 g, or 3 g, preferably at least 4 g, in and it may be divided over one or more servings. It may even go up to a daily intake of 10g, 20g or 30 g divided over one or more servings.

It further varies depending on the kind and form of the food, pet food, food supplement, feed and/or beverages. It further may depend upon characteristics of the subject, human, or animal (pet, horses, fish, aqua- animals in general or any other monogastric farm animal, and poultry), and person related characteristics such as age and body weight.

The foods, pet foods, feed and beverages useable in the current invention are not particularly limited. When these foods pet foods, feed and beverages are taken the functions of the present invention are exhibited.

The foods, pet foods, feed and beverages of the current invention are compositions intended to be ingested by humans and animals and refer to compositions or products intended to be ingested and administered orally or enterally. Suitable foods may encompass confectionery, pastes to be chewed, chewing gums, pastries, cakes, oily cakes, snacks, bars, ice creams, sorbets, chilled desserts, preparations based on milk (dairy or non-dairy such as soymilk, coconut milk and the like), yoghurts, jams, soups, processed farm products, seasonings, canned foods, processed meats, processed marine products, foodstuffs for animals (non-ruminants (i.e. pet, horses, aqua-animals, poultry, monogastric animals)), pharmaceutical products, food supplements, veterinary, dietetic or hygiene products such as for example elixirs, cough syrups, tablets or pills, hygienic solutions for oral cavity, toothpastes and tooth gels, and the like. Suitable drinks (beverages) may include solid or liquid drinks. The beverage can be any drinkable solution including iced tea, and fruit juices, vegetable based juices, lemonades, cordials, nut based drinks, cocoa based drinks, dairy products such as milk, whey, yogurts and drinks based on them, non-dairy milk based drinks, such as coconut milk, soymilk, almond milk and the like.

Solid drinks refer to beverage concentrates, smoothies, and drink powders. Beverage concentrates refer to concentrates that is either in liquid form or in essentially dry mixture form. The liquid concentrate can be in the form of a relatively thick, syrupy liquid. The essentially dry mixture can be in the form of either a powder or a tablet. The beverage concentrate is usually formulated to provide a drinkable beverage composition or a final beverage when constituted or diluted with water, either carbonated or non-carbonated.

Drink powders are suitable for constituting with water, carbonated or non-carbonated, a final beverage for oral administration of fiber source of current invention. Further specific beverages may include hypotonic drinks, sports drinks, hypertonic drinks, energy drinks, isotonic drinks, flavoured and/or vitaminized water and the like.

The food and beverages can also be considered suitable as diabetic foods, baby food, infant food, dietetic food, slimming food, food for special dietary needs and medical food. Medical food refers to any liquid, semi-solid or liquid comestible which is given to people in medical need for having access to extra fiber containing materials.

The foods, pet foods, feed and beverages further may comprise carbohydrates different from the carbohydrate mixture, proteins, peptides, amino acids, antioxidants, fats, vitamins, trace elements, electrolytes, non-caloric high intensity sweeteners, flavours and/or mixtures thereof. These carbohydrates are selected from the group consisting of monosaccharides, disaccharides, gelling starches, starch hydrolysates, viscous fiber containing materials, any other hydrocolloid material (for example xanthan gum, carrageenan, pectin and the like), cellulosic materials, hemicellulosic materials, polyols and mixtures of two or more thereof.

The monosaccharides include tetroses, pentoses, hexoses and ketohexoses. Starch hydrolysates are produced by the controlled acid or enzymatic hydrolysis of starch and can be subdivided into two specific categories, maltodextrins and glucose syrups and are characterized by DE number (dextrose equivalent). In fact, DE number is a measurement of the percentage of reducing sugars present in the syrup and calculated as dextrose on a dry weight basis. Maltodextrins have a DE number up to 20 whereas glucose syrups have an DE number greater than 20. Gelly starches may include emulsified starches such as starch n-octenyl succinate.

The polyol can be selected from tetritols, pentitols, hexitols, and higher polyols. The polyol may include but is not limited to erythritol, xylitol, arabinitol, sorbitol, mannitol, iditol, galactitol, maltitol, isomaltitol, isomalt, lactitol, mixtures of two or more thereof, and the like. Preferably the polyol is erythritol, mannitol, isomalt, maltitol or a mixture of two or more thereof.

A non-caloric high intensity sweetener, which can be used as non-nutritive sweetener can be selected from the group consisting of aspartame, acesulfame salts such as acesulfame-K, saccharins (e.g. sodium and calcium salts), cyclamates (e.g. sodium and calcium salts), sucralose, alitame, neotame, steviosides, glycyrrhizin, neohesperidin, thaumatin, brazzein and mixtures of two or more thereof, preferably stevioside and the like.

Among the major physiological electrolytes are sodium, potassium, chloride, calcium, and magnesium. Further trace elements can be included such as chromium, copper, selenium, iron, manganese, molybdenym, zinc and mixtures thereof.

Among the vitamins one can range vitamin A, vitamin C, vitamin E and/or vitamin B12.

The amount of flavor depends upon the flavor or flavors selected, the flavor impression desired and the form of flavor used. If desired, coloring agents can also be added. Any soluble coloring agent approved for food use can be utilized for the current invention.

The carbohydrate mixture of the current invention is used in composition intended to be ingested by humans and animals (such as foods, drinks (beverages), pet food, feed, pharmaceutical compositions, food supplements).

In particular it is used for promoting the growth of health beneficial gut microbiota, such as bifidobacteria and lactobacilli.

The promotion of said microbiota has substantial health promoting effects, such as reduced constipation, and/or reduced fermentation of proteins, and/or thus reduced risks of colon cancer.

These health benefits (=promotion of the growth of health beneficial gut microbiota, such as bifidobacteria and lactobacilli) are provided by an effective amount of the carbohydrate mixture in food, food supplements, pet food, feed and/or beverages.

The consumption of the foods and beverages containing the carbohydrate mixture of the current invention should be such that the daily intake of the carbohydrate mixture of the current invention is at least amounting in total to 1 g, preferably 1.5 g, preferably to at least 2.5g, 3 g, preferably at least 4 g, in particular significant for the use by humans, and divided over one or more servings. It may even go up to a daily intake of 10g, 20g or 30 g divided over one or more servings. As is mentioned before, it further may depend upon characteristics of the subject, human, or animal (pet, horses, fish, aqua- animals in general or any other monogastric farm animal, and poultry), and person related characteristics such as age and body weight.

The current invention presents the following advantages:
- The carbohydrate mixture can be applied in solid, semi-solid and liquid comestibles.
- The carbohydrate mixture can provide viscosity and consequently satiety effect.
- It is suitable for a regular diet, for athletic food, diabetic food, baby food, infant food, food for elderly people and those requiring a special diet in respect of fiber containing materials.
- It is suitable for pet food and animal nutrition (non-ruminant).
- It is providing significant health benefits, more in particular it is reducing constipation.

### Methods of measurement

### Average DP and A/X ratio of AXOS composition

AXOS preparations are mixtures of structurally different components. Gas chromatographic analysis allows revealing structural information on these complex preparations. However, one must keep in mind that the determined parameters are average values, and provide information about the mixture of the structurally different components.

Measurement of:
- Total arabinose content and total xylose content
- Reducing end xylose content
- Free arabinose content and free xylose content

Hydrolysis is done with 2.0 M trifluoroacetic acid at 110 °C for 60 min.
Reduction is done with sodium borohydride.
Acetylation is done in presence of acetic anhydride.

The total arabinose content and the total xylose content of AXOS preparations is determined by gas chromatography of alditol acetates obtained after acid hydrolysis and reduction and acetylation (Englyst H.N. and Cummings J.H. 1984. Simplified method for the measurement of total non-starch polysaccharides by gas - liquid-chromatography of constituent sugars as alditol acetates. Analyst 109(7): 937-942). Samples (approximately 10-20 mg accurately weighed) are hydrolysed with 2.0 M trifluoroacetic acid (5.0 mL) at 110 °C for 60 min. The resulting monosaccharides are reduced with sodium borohydride and converted to alditol acetates. For identification and quantification, an internal standard (D-allose) and calibration samples are used.

For determination of the content of reducing end xylose, samples (20-30 mg dissolved in 2.5 mL of H₂O) are reduced prior to hydrolysis followed by acetylation to alditol acetates (Courtin C.M., Van den Broeck H. and Delcour J.A. 2000. Determination of reducing end sugar residues in oligo- and polysaccharides by gas-liquid chromatography. Journal of Chromatography A 866(1): 97-104).

For analysis of free arabinose content and free xylose content, samples are reduced followed by acetylation.

The AXOS content is calculated as the sum of the total contents of xylose and arabinose multiplied by 0.88.

The A/X ratio is calculated as the total arabinose content divided by the total xylose content. The average DP is calculated as the sum of the total xylose content and total arabinose content, whereby the sum is divided by the content of reducing end xylose.

In each of the previous calculations the content of free arabinose and/or xylose needs to be subtracted from the total arabinose and/or xylose content.

### Molecular weight distribution of AXOS

Molecular weight distribution of AXOS preparations is determined by High Performance Size Exclusion Chromatography (Courtin C.M., Van den Broeck H. and Delcour J.A. 2000. Determination of reducing end sugar residues in oligo- and polysaccharides by gas-liquid chromatography. Journal of Chromatography A 866(1): 97-104). Apparent molecular weights can be estimated based on retention times of pullulan standards.

### Ferulic acid content

Total ferulic acid content is determined by reversed phase HPLC after saponification and extraction of the ferulic acids (Antoine C., Peyron S., Mabille F., Lapierre C., Bouchet B., Abecassis J. and Rouau X. 2003. Individual contribution of grain outer layers and their cell wall structure to the mechanical properties of wheat bran. Journal of Agricultural and Food Chemistry 51 (7): 2026-2033)

## Claims

1. A carbohydrate mixture comprising a first fiber containing material and a second fiber containing material wherein the first fiber containing material is wheat bran derived arabinoxylan-oligosaccharides having an average degree of polymerisation of at least 2 to 100 and optionally wheat bran derived xylo-oligosaccharides having an average degree of polymerisation of at least 2 to 100, and wherein the second fiber containing carbohydrate based material is endosperm derived xylo-oligosaccharides and arabinoxylan-oligosaccharides having an average degree of polymerisation of at least 50 to 100 and/or hydrogenated endosperm derived xylo-oligosaccharides and arabinoxylan-oligosaccharides having an average degree of polymerisation of at least 50 to 100.

2. The carbohydrate mixture according to claim 1 wherein the wheat bran derived arabinoxylan-oligosaccharides and optionally wheat bran derived xylo-oligosaccharides, have an average degree of polymerisation of from 2 to 50.

3. The carbohydrate mixture according to claim 1 wherein the first fiber containing material is present in an amount of from 5 to 95% based on dry matter content of carbohydrate mixture.

4. The carbohydrate mixture according to claim 1 wherein the first fiber containing material is present in an amount of from 25 to 75% based on dry matter content of carbohydrate mixture.

5. The carbohydrate mixture according to claim 1 wherein the first fiber containing material is present in an amount of from 40 to 60% based on dry matter content of carbohydrate mixture.

6. Use of carbohydrate mixture according to claim 1 for promoting growth of health beneficial gut microbiota.

7. The use according to claim 6 wherein constipation is reduced.

8. The use according to claim 6 or 7 wherein the promotion is provided by an effective amount of carbohydrate composition according to claim 1 in compositions intended to be ingested by humans and animals.

9. The use according to claim 8 wherein the compositions intended to be ingested by humans and animals is food, food supplements, pet food, feed, and/or beverages.
